# EUROPEAN PATENT APPLICATION

(11) **EP 3 871 519 A1**
(43) Date of publication of application: **01.09.2021**
(21) Application number: 21154281.6
(22) Date of filing: 15.12.2016
(51) Int. Cl.: A24F 40/40, A24F 40/60, A61M 15/00, A61M 15/06, A61M 11/04, A61M 16/00, A24F 40/10, A24F 40/42

(54) **PERSONAL VAPORIZER DEVICE**

(30) Priority: 18.12.2015 EP 15201281
(62) Divisional of application: 16809444.9
(71) Applicant: JT International S.A., 1202 Geneva (CH)
(72) Inventor: JAMES, Aled, Dorridge B93 8RR (GB); THOMAS, Richard, Leamington Spa CV32 4SA (GB); MAY, James, Kenilworth CV8 1BS (GB)
(74) Representative: Hoffmann Eitle

(57) **Abstract**

The invention provides a personal vaporizer device (1), especially an electronic smoking article. The personal vaporizer device (1) comprises a cartridge (2) which includes a reservoir (8) for storing a liquid to be vaporized; and an elongate body portion (4) to which the cartridge (2) is connected. The elongate body portion (4) comprises a cover member (6) which is movable in a longitudinal direction of the body portion (4) between a first position (A) and a second position (B), wherein the cartridge (2) is at least partially covered or obscured by the cover member (6) in the first position (A). In the second position (B), the cover member (5) is retracted.

## Description

### Field of the Invention

The present invention relates to a cartridge and a personal vaporizer device, such as an electronic smoking article.

### Background of the Invention

Personal vaporizer devices, such as electronic cigarettes or "e-cigarettes" as they are also known, have gained in popularity over the past ten years as an alternative to traditional smoking articles, like cigarettes, cigars, and cigarillos. Because the technology employed in personal vaporizer devices is still quite young, however, developments in the design and configuration of such devices are on-going to improve their performance and their reliability, as well as their ease of use, ease of production, and their production costs.

### Summary of the Invention

In view of the above, an object of the invention is to provide a new and improved cartridge and personal vaporizer device, especially an improved electronic smoking article. In particular, it would be desirable to provide such a personal vaporizer device which is more ergonomic and user-friendly for a user. It would also be useful to provide such a personal vaporizer device which protects sensitive or replaceable parts or components of the device, such as a cartridge or capsule for holding a liquid to be vaporized, especially a disposable cartridge or a refillable cartridge.

In accordance with the present invention, a personal vaporizer device, especially an electronic smoking article, as recited in claim 1 is provided. Various preferred and/or advantageous features of the invention are recited in the dependent claims. According to one aspect, therefore, the present invention provides a personal vaporizer device, especially an electronic smoking article, configured to receive a removable cartridge including a reservoir for storing a liquid to be vaporized, the vaporizer device comprising: an elongate body portion to which the cartridge is configured to be connected. The elongate body portion comprises a cover member which is movable in a longitudinal direction of the body portion between a first position and a second position, and the cover member is configured and arranged to cover or obscure the cartridge at least partially in the first position. In this way, when in the first position the cover member is able to protect the cartridge from external influences, and especially from physical forces or impacts.

According to another aspect, the invention provides a personal vaporizer device, especially an electronic smoking article, comprising: a cartridge which includes a reservoir for storing a liquid to be vaporized; and an elongate body portion to which the cartridge is connected. The elongate body portion includes a cover member which is movable in a longitudinal direction of the body portion between a first position and a second position, wherein the cartridge is at least partially, and optionally substantially entirely, covered or obscured by the cover member in the first position. As noted above, the cover member may thus provide a physical barrier for protecting the cartridge in the first position.

In relation to the cartridge being at least partially covered or obscured by the cover member in the first position, it will be noted that the cover member typically covers or obscures at least 50% of an outer surface of the cartridge in the first position, and more preferably at least 80% of an outer surface of the cartridge in the first position. In a preferred embodiment, the cover member covers or obscures in the range of 80% to 100% of the cartridge in the first position.

In a preferred embodiment, the cover member may substantially cover or obscure a mouthpiece of the personal vaporizer device in the first position. In this way, the first position may be clearly designed or intended to form a non-use position or a non-activated position for the vaporizer device. The mouthpiece may, for example, be provided on the cartridge. In this context, the first position of the cover member may be a substantially extended position, in which the cover member extends in the longitudinal direction of the body portion to cover the cartridge and/or mouthpiece of the personal vaporizer device.

In a preferred embodiment, the cartridge is configured to be connected to an end region of the elongate body portion, wherein the cover member comprises at least one of a front cover panel, which extends over a front of the elongate body portion, and a rear cover panel, which extends over a rear of the elongate body portion. In this way, the cover member may form part of an outer casing of the elongate body portion.

In a preferred embodiment, a volume of liquid in the reservoir of the cartridge is or remains visible when the cover member is in the first position. That is, although the cartridge is largely covered or obscured by the cover member in this position, a user is nevertheless able to check and see how much liquid is in the reservoir of the cartridge. In this regard, a window could be provided in the cover member. As an alternative, or in addition, the cover member need not fully enclose or encase the cartridge, thereby leaving an area or region uncovered for a user to determine visually what volume of liquid remains in the reservoir. For example, a side region of the cartridge could optionally remain uncovered by the cover member.

In a preferred embodiment, the second position of the cover member may assume a substantially retracted position, with the cover member configured and arranged to leave the cartridge substantially uncovered or unobscured in the second position. That is, the cover member may be retracted from the cartridge in the longitudinal direction to the second position. Thus, this second position may be designed or intended to form a use position or an activated position for the vaporizer device. Further, the second position may be particularly suitable for connecting a cartridge to and/or for removing a cartridge from the body portion of the personal vaporizer device.

In a preferred embodiment, the personal vaporizer device comprises guide means for guiding movement of the cover member in the longitudinal direction between the first position and the second position. In this regard, the cover member may, for example, be mounted on one or more tracks or rails provided in or on the body portion for movement of the cover member in the longitudinal direction between the first position and the second position. That is, the one or more tracks or rails may define a path of travel for the cover member between the first position and the second position. The cover member therefore preferably includes one or more complementary follower element configured for engaging a respective track or rail for following same along the predefined path of travel between the first and second positions. The movement of the cover member in the longitudinal direction may be a sliding movement, although rolling movement or other translational movement is also contemplated.

In a preferred embodiment, the elongate body portion includes a switch which is configured to activate one or more functionality of the personal vaporizer device. In this regard, the switch is preferably provided either on the movable cover member or in operative connection with the cover member. In this way, the one or more functionality of the device may, for example, be activated via the switch when the cover member is moved to the first position and/or to the second position. For example, the device may include two separate functions which could be activated by movement of the cover member, with one function being activated when the cover member is in the first position, and another function being activated when the cover member is in the second position. Alternatively, or in addition, multiple functions could be activated in each of the first and/or second positions.

In a preferred embodiment, the elongate body portion comprises a power supply unit, especially a battery unit, for supplying electrical power to the device. In this regard, the personal vaporizer device will typically include a vaporizer unit for vaporizing the liquid from the reservoir to be inhaled by a user. Furthermore, the vaporizer device may optionally include a control unit for controlling operation of the device. Thus, the power supply unit is desirably designed to supply electrical power to the vaporizer unit and/or to the control unit. In a particularly preferred embodiment, the vaporizer unit and/or the control unit may be incorporated in the cartridge which is configured to be connected to the elongate body portion of the device. Accordingly, in a preferred embodiment the cartridge comprises a housing that encloses the reservoir for storing the liquid to be vaporized. The vaporizer unit comprises a heater for heating the liquid to be vaporized to generate the vapour to be inhaled, and a liquid delivery means which is configured to convey the liquid from the reservoir to the heater for vaporization.

As noted above, the cartridge is preferably configured to be connected to an end region of the elongate body portion in the vaporizer device. The end region of the elongate body portion thus preferably includes one or more electrical connectors for making an electrical connection with one or more complementary electrical connectors provided on the cartridge.

Furthermore, in a preferred embodiment, the body portion has one or more indicator, especially an illuminated indicator, such as an LED, for indicating an operational status of the device and/or for indicating available power supply in a power supply unit of the elongate body portion.

According to a further aspect, the present invention provides a personal vaporizer device, especially an electronic smoking article, configured to receive a removable cartridge with a reservoir for holding a liquid to be vaporized. The vaporizer device comprises an elongate body portion to which the cartridge is configured to be connected. The elongate body portion includes a power supply unit, especially a battery unit, for supplying electrical power to the device and at least one part of a controller or control unit for controlling operation of the device. A user interface for the control unit or controller is provided on an outer cover member of the elongate body portion and includes one or more user-interface elements over a longitudinal extent of the elongate body portion.

In a preferred embodiment, the control unit or controller is configured to manage one or more of: a heater or heating function of the device; a power supply function of the device; and a capsule recognition function of the device. The control unit or controller typically comprises a user interface for the device. Preferably, one or more LEDs are provided to act or to serve as illuminated indicator elements for indicating an operational status of the device and/or for indicating available power supply in the power supply unit.

In a preferred embodiment, the personal vaporizer device comprises one or more sensors for determining one or more operating condition of the device. The sensor or sensors may be incorporated in the cartridge and/or in the body portion of the device. The sensor(s) may, for example, include a temperature sensor, a liquid volume sensor, a puff sensor, and/or gyroscopic sensors.

According to a further aspect, the invention also provides a method of installing a cartridge in a personal vaporizer device, comprising the steps of:
providing a personal vaporizer device having an elongate body portion to which a removable cartridge is configured to be connected, the cartridge including a reservoir for storing a liquid to be vaporized;
providing a cover member on the elongate body portion which is movable in a longitudinal direction of the body portion between a first extended position and a second retracted position;
moving the cover member to the second position to access an end region of the body portion; and
attaching the removable cartridge to the accessed end region of the body portion personal vaporizer device.

In an embodiment of the method, after attaching the cartridge to the end region of the body portion, the cover member is moved to the first position to substantially cover the cartridge. As noted above, this has the effect of protecting the installed cartridge from external influences.

In an embodiment of the method, after attaching the cartridge to the end region of the body portion, movement of the cover member between the first position and the second position may act to switch the personal vaporizer device between an activated and a deactivated state.

### Brief Description of the Drawings

For a more complete understanding of the invention and the advantages thereof, exemplary embodiments of the invention are explained in more detail in the following description with reference to the accompanying drawing figures, in which like reference characters designate like parts and in which:
- Fig. 1: is a side view of a personal vaporizer device, especially an electronic smoking article, according to an embodiment, with a cover member of the device shown in a first position;
- Fig. 2: is a side view of the personal vaporizer device shown in Fig. 1, with the cover member of the device shown in a first position;
- Fig. 3: is a perspective view of a cartridge for a personal vaporizer device according to an embodiment;
- Fig. 4: is a schematic sectioned side view of a cartridge with vaporizer unit installed in a personal vaporizer device via a first type of connection;
- Fig. 5: is a schematic sectioned side view of a cartridge with vaporizer unit installed in a personal vaporizer device via a second type of connection;
- Fig. 6: is a schematic sectioned side view of a cartridge with vaporizer unit installed in a personal vaporizer device via a third type of connection;

- Fig. 7: is a perspective view of the personal vaporizer device illustrated in Figs. 1 and 2, with the cover member shown in the first position;
- Fig. 8: is a perspective view of the cover member of the personal vaporizer device shown in Figs. 1, 2 and 7;
- Fig. 9: is a perspective view of an underside of the cover member shown in Fig. 8;
- Fig. 10: is a perspective view of the personal vaporizer device shown in Fig. 7 with the cover member represented transparent providing a view of the control unit and user interface elements;
- Fig. 11: is a flow diagram which schematically represents a method according to an embodiment of the invention.

The accompanying drawings are included to provide a further understanding of the present invention and are incorporated in and constitute a part of this specification. The drawings illustrate particular embodiments of the invention and together with the description serve to explain the principles of the invention. Other embodiments of the invention and many of the attendant advantages of the invention will be readily appreciated as they become better understood with reference to the following detailed description.

It will be appreciated that common and/or well understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

### Detailed Description of Embodiments

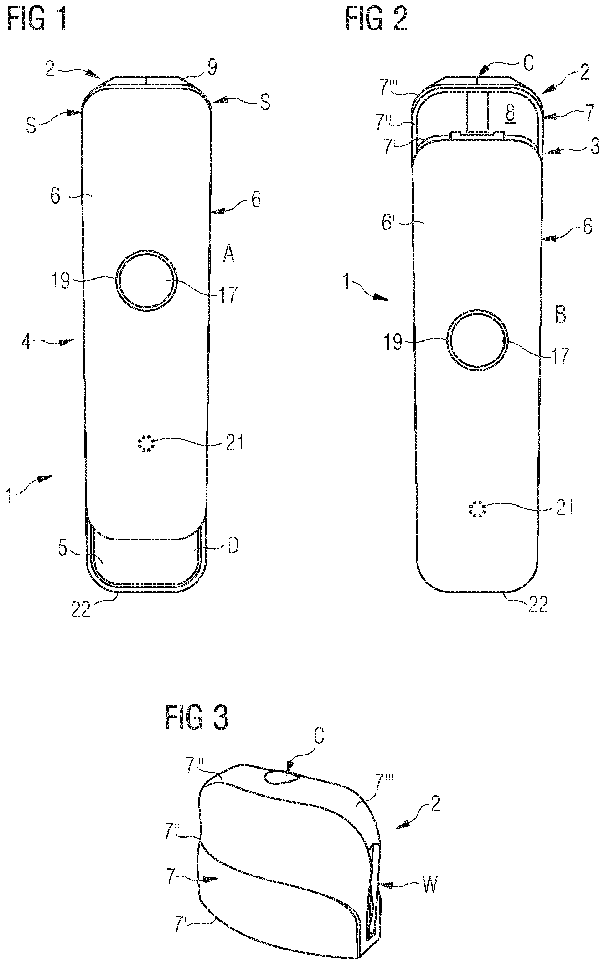

With reference firstly to Figs. 1 and 2 of the drawings, a personal vaporizer device 1 in the form of electronic cigarette or "e-cigarette" is illustrated in different positions and in partial cross-section. The device 1 includes a replaceable cartridge 2 which is attached at one end region 3 of an elongate body 4. The elongate body 4 in turn comprises a casing 5 which encloses a power supply unit in the form of a battery unit D for supplying electrical power to the device 1. The casing 5 of the elongate body 4 includes a cover member 6 which is movable in a longitudinal direction of the body 4 between a first position A (i.e. shown in Fig. 1) and a second position B (i.e. shown in Fig. 2). In this regard, the cover member 6 comprises a front cover panel 6' which extends over a front side of the body 4, and a rear cover panel 6", which extends over a rear side of the body 4.

As can be seen in drawing Figs. 2, the cartridge 2 comprises a housing 7 that encloses a reservoir 8 for storing a liquid to be vaporized. The housing 7 has a flat base 7' and generally vertically extending sides 7" which transition at curved upper shoulder regions 7''' to form or terminate at a mouthpiece 9 at a top of the cartridge 2. In this way, the shell of the housing 7 encloses the reservoir 8 to form a small tank for storing the liquid (i.e. of a type known in the art) to be vaporized for inhalation by a user of the e-cigarette. Furthermore, the cartridge 2 includes an airflow channel C which extends centrally through the housing 7 from the base 7' to the top and is configured to guide the vapour to the mouthpiece 9 for inhalation by the user. The airflow channel C has a generally circular cross-section and is surrounded by the reservoir 8. In order to generate the vapour, the cartridge 2 incorporates a vaporizer unit having an electric heater with a heating element or wire provided wound in a coil within the airflow channel C for heating the liquid to be vaporized to generate the vapour to be inhaled. The vaporizer unit (not shown) further comprises a liquid delivery means which is configured to convey the liquid from the reservoir 8 to the heating wire for vaporization.

Referring to drawing Figs. 1 and 2, the cover member 6, which is comprised of the front and rear panel members 6', 6", is movable in a longitudinal direction of the device body 4 - e.g. by sliding or in translation - between the first position A shown in Fig. 1 and the second position B shown in Fig. 2. In the first extended position A, the replaceable cartridge 2 comprising the reservoir 8 and the vaporizer unit 10 is substantially covered or obscured by the cover member 6. In this way, the cover member 6 provides a physical barrier for protecting the cartridge 2 in the first position A. Nevertheless, the volume or amount of liquid in the reservoir 8 of the cartridge 2 remains visible when the cover member 6 is in the first position A. That is, although the cartridge 2 is largely covered or obscured by cover member 6 in this position, a window may be provided in the cover member 6 and/or a side region S of the cartridge 2 remains uncovered by the cover member 6 and may include a window W for a user to determine visually how much liquid remains in the reservoir 8.

For sliding or moving the cover member 6 relative to the remainder of the casing 5 between the first and second positions A, B, at least one or both of the front and rear panel members 6', 6" may be held and engaged between a thumb and finger of the user. In the second position B, the cover member 6 is retracted to leave the cartridge 2 either partially or substantially uncovered or unobscured. That is, in the second position B the cover member 6 is retracted from the cartridge 2 in the longitudinal direction to a use position or an activated position for the e-cigarette or vaporizer device 1. To this end, the body 4 includes a switch 17 provided in the form of a round button set within an opening 18 in the cover member 6. The switch or button 17 is configured to activate the device 1 and the switch 17 is in operative connection with a part of the controller or control unit below the cover member 6. In this regard, the device 1 may be designed to only be able to be activated via the switch 17 when the front and rear panels 6', 6" are moved from the first position A to the second position B. The switch 17 is surrounded by a ring-shaped indicator 19 which illuminates on the front panel 6' for indicating an operational status of the device 1. These elements of the vaporizer device 1 will be described in greater detail below.

In addition, the second position B of the cover member 6 is designed for removing the cartridge 2 from, and/or for connecting a cartridge 2 to, the body portion 4 of the personal vaporizer device or e-cigarette 1. As noted above, the cartridge 2 is configured to be connected to the end region 3 of the body portion 4. This end region 3 of the elongate body 4 thus preferably includes one or more electrical contacts 14 for making an electrical connection with one or more complementary electrical connectors provided on the cartridge 2 for connecting electrical power to the heater of the vaporizer unit within the cartridge 2.

Drawing Fig. 3 shows a perspective view of a cartridge 2 according to a similar embodiment to the one described above. As described previously, the cartridge 2 here typically includes a housing 7 enclosing a reservoir 8 for storing the liquid to be vaporized and defining an airflow channel C which extends centrally through the housing 7 to a mouthpiece 9 at a top of the cartridge 2. Further, the cartridge 2 includes a vaporizer unit (not shown) having an electric heater with a heating element provided wound in a coil within the airflow channel C for heating the liquid to be vaporized to generate the vapour to be inhaled. The vaporizer unit comprises a liquid delivery means configured to convey the liquid from the reservoir 8 via capillary action or other known mechanisms.

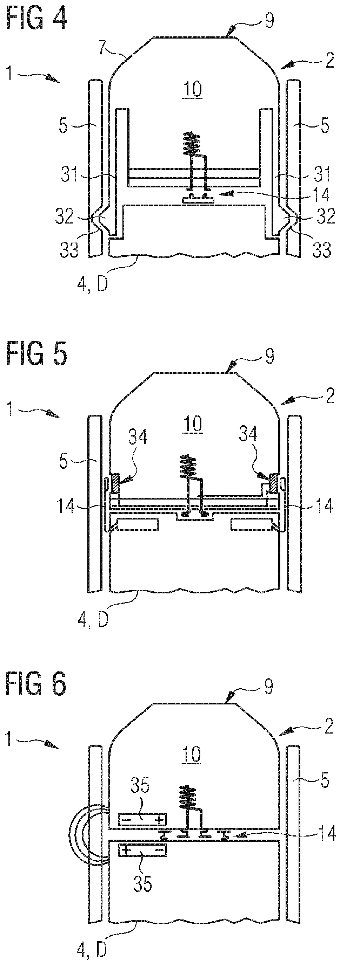

Referring now to Figs. 4 to 6 of the drawings, three different types of mechanical and electrical connections between the cartridge 2 and power supply (e.g. battery unit D) via the end region 3 of the body 4 of the personal vaporizer device 1 are schematically illustrated. In each case, the cartridge 2 is configured for a push-on/pull-off type connection into the open upper end region 3 of the casing 5 of the device. In Fig. 4, the sides 7" of the cartridge housing 7 include resilient or flexible members 31 (e.g. formed as leaf-springs or cantilevers) having projecting lugs 32 for receipt or engagement in complementary recesses 33 formed on an inner side of the casing 5. Electrical contact elements 14 are provided separately for power supply to the controller 13 and heater via electrical contacts provided below the cartridge 2. In the embodiment of Fig. 5, the sides 7" of the cartridge housing 7 are received in the open upper end region 3 of the casing 5 and resilient electrical contact elements 14 extend upwards from the power supply and engage electrical contacts 34 provided in the sides 7" of the cartridge housing 7. In the embodiment of Fig. 6, by contrast, a mechanical connection is effected via magnetic connector elements 35 provided on both the cartridge 2 and the body part 4 of the personal vaporizer device 1 at the end region 3. The electrical connection again occurs separately via electrical contacts 14 below the base 7' of the cartridge housing 7.

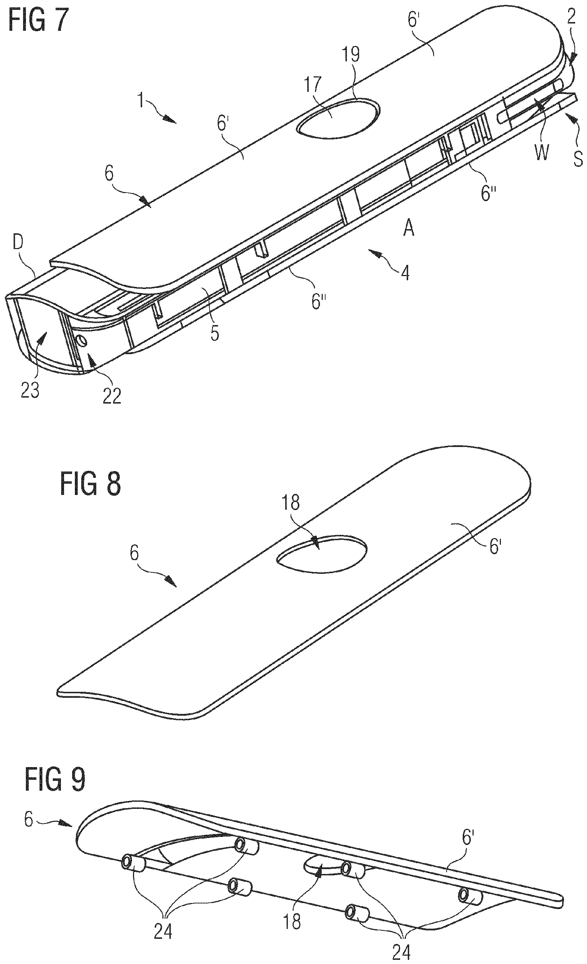

With reference now to Figs. 7 to 9 of the drawings, the geometry and construction of the cover member 6 can be more clearly seen. In particular, the front panel 6' of the cover member is functionalized by its convex outer shape, its thickness and flexibility. In particular, the panel 6' is not only configured to move between the first and second positions A, B but may also bend and form as a user interface for operation of the device 1. In this regard, it has already been noted above that the front panel 6' of the cover member 6 includes user interface elements, such as the switch 17 in the form of a button set within the opening 18 in the cover member 6 and surrounded by a ring-shaped indicator 19 which illuminates the front panel 6' for indicating an operational status of the device 1. The switch or button 17 which is configured to activate the device 1 for vapour generation after the cover member 6 has been moved to the second or "in-use" position B. The front panel 6' may also include one or more further switch or button 21 which may be positioned on a surface thereof (e.g. discretely or clearly) for activating additional functionalities, such as for setting an operating mode. Each of the switches or buttons 17, 21 is in operative connection with a part of the controller or control unit 13 of the device 1 below the cover member 6, which is described later. When the cover member 6 is moved to the second position B, the indicator 17 may illuminate to identify that the device 1 has been activated and/or is ready for operation. Further, the indicator 20 may illuminate in a different colour and/or in a different manner (e.g. blink or pulsate) to signal to a user that the available power supply in the battery unit D is low (e.g. that the battery unit D should be recharged). For this purpose, the opposite end region 22 of the body 4 of the e-cigarette 1 typically includes a connector 23 for connection to a re-charging dock or re-charging station and/or for connection of a re-charging cable.

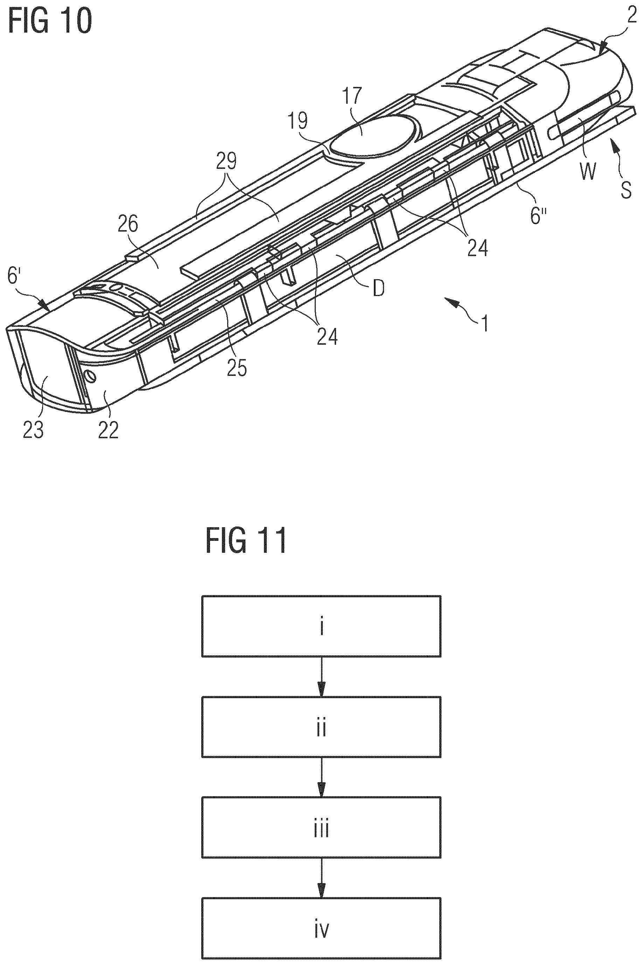

As is apparent from drawing Fig. 9, the underside of the front panel 6' includes sleeve elements 24 for sliding engagement with parallel rail members 25 (seen in Fig. 10) on the body portion 4 during longitudinal movement between the first and second positions A, B. In this way, the rail members 25 function as guide means for guiding the longitudinal movement of the cover member 6 and sleeve elements 24 form follower elements for following the path defined by the rails. Further, referring to drawing Fig. 10, the elongate body portion 4 of the device 1 includes a circuit board (not shown) mounted below the front panel 6' which forms part of the controller or control unit 13 of the e-cigarette.

Finally, referring to Fig. 11 of the drawings, a flow diagram is shown that illustrates schematically the steps in a method of installing a cartridge in a personal vaporizer device 1, especially in an electronic smoking article according to the embodiments of the invention described above with respect to Figs. 1 to 10. In this regard, the first box i of Fig. 11 thus represents the step of providing a personal vaporizer device 1 having an elongate body portion 4 to which a removable cartridge 2 is configured to be connected, the cartridge 2 including a reservoir 8 for storing a liquid to be vaporized. The second box ii represents the step of providing a cover member 6 on the elongate body portion 4 which is movable in a longitudinal direction of the body portion 4 between a first extended position A and a second retracted position B. The third box iii represents the step of moving cover member 6 to the second retracted position B to access an end region 3 of the body portion 4. The final box iv in Fig. 11 of the drawings represents the step of attaching the cartridge 2 to the accessed end region 3 of the body portion 4 personal vaporizer device 1.

Although specific embodiments of the invention are illustrated and described herein, it will be appreciated by those of ordinary skill in the art that a variety of alternate and/or equivalent implementations exist. It should be appreciated that the exemplary embodiment or exemplary embodiments are examples only and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents. Generally, this application is intended to cover any adaptations or variations of the specific embodiments discussed herein.

It will also be appreciated that in this document the terms "comprise", "comprising", "include", "including", "contain", "containing", "have", "having", and any variations thereof, are intended to be understood in an inclusive (i.e. non-exclusive) sense, such that the process, method, device, apparatus or system described herein is not limited to those features or parts or elements or steps recited but may include other elements, features, parts or steps not expressly listed or inherent to such process, method, article, or apparatus. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

### Further Embodiments

1. A personal vaporizer device (1), especially an electronic smoking article, comprising:
   a cartridge (2) which includes a reservoir (8) for storing a liquid to be vaporized; and
   an elongate body portion (4) to which the cartridge (2) is connected,
   wherein the elongate body portion (4) comprises a cover member (6) which is movable in a longitudinal direction of the body portion (4) between a first position (A) and a second position (B),
      wherein the cartridge (2) is at least partially covered or obscured by the cover member (6) in the first position (A).
2. A personal vaporizer device (1) according to 1, wherein a volume of liquid in the reservoir (8) of the cartridge (2) remains visible when the cover member (6) is in the first position (A).
3. A personal vaporizer device (1) according to 1 or 2, wherein the second position (B) of the cover member (6) is a substantially retracted position in which the cartridge (2) is substantially uncovered or unobscured by the cover member (6), especially for connection of the cartridge (2) to, and/or removal of the cartridge (2) from, the elongate body portion (4).
4. A personal vaporizer device (1) according to any one of 1 to 3, wherein the first position (A) of the cover member (6) is a substantially extended position.
5. A personal vaporizer device (1) according to any one of 1 to 4, wherein the cover member (6) forms a part of an outer casing (5) of the elongate body portion (4).
6. A personal vaporizer device (1) according to 5, wherein the cartridge (2) is configured to be connected to an end region (3) of the elongate body portion (4), wherein the cover member (6) comprises at least one of a front cover panel (6') which extends over a front of the elongate body portion (4) and a rear cover panel (6") which extends over a rear of the elongate body portion (4).
7. A personal vaporizer device (1), especially an electronic smoking article, configured to receive a removable cartridge (2) including a reservoir (8) for storing a liquid to be vaporized, the vaporizer device (1) comprising:
   an elongate body portion (4) to which the cartridge (2) is configured to be connected, wherein the elongate body portion (4) comprises a cover member (6) which is movable in a longitudinal direction of the body portion (4) between a first position (A) and a second position (B),
   wherein the cover member (6) is configured and arranged to cover or obscure the cartridge (2), at least partially, in the first position (A).
8. A personal vaporizer device (1) according to 7, wherein the second position (B) of the cover member (6) is a substantially retracted position, the cover member (6) being configured and arranged to leave the cartridge (2) substantially uncovered or unobscured in the second position (B), especially for connection of the cartridge (2) to, and/or removal of the cartridge (2) from, the elongate body portion (4).
9. A personal vaporizer device (1) according to any one of 1 to 8, wherein the elongate body portion (4) comprises a power supply unit (D), especially a battery unit, for supplying electrical power to the cartridge (2) when it is connected to the elongate body portion (4).
10. A personal vaporizer device (1) according to 9, wherein the cartridge (2) is configured to be connected to an end region (3) of the elongate body portion (4), and wherein the end region (3) of the elongate body portion (4) comprises electrical connectors (14) for making an electrical connection with complementary electrical connectors provided on the cartridge (2).
11. A personal vaporizer device (1) according to any one of 1 to 10, wherein the elongate body portion (4) has one more indicator (17), such as an LED, for indicating an operational status of the device (1) and/or for indicating available power supply in a power supply unit (D) of the elongate body portion (4).
12. A personal vaporizer device (1) according to any one of 1 to 11, wherein the elongate body portion (4) comprises a switch for activating one or more functionalities of the device (1), the switch preferably being provided on or operatively connected with the cover member (6), wherein the one or more functionalities of the device (1) is activated when the cover member (6) is in the second position (B).
13. A method of installing a cartridge (2) in a personal vaporizer device (1), and especially in an electronic smoking article, comprising the steps of:
   providing a personal vaporizer device (1) having an elongate body portion (4) to which a removable cartridge (2) is configured to be connected, the cartridge (2) including a reservoir (8) for storing a liquid to be vaporized;
   providing a cover member (6) on the elongate body portion (4) which is movable in a longitudinal direction of the body portion (4) between a first extended position (A) and a second retracted position (B);
   moving cover member (6) to the second position (B) to access an end region (3) of the body portion (4); and
   attaching the removable cartridge (2) to the accessed end region (3) of the body portion (4) personal vaporizer device (1).
14. A method according to 13, comprising the step of: after attaching the cartridge (2) to the end region (3) of the body portion (4), moving the cover member (6) to the first position (A) to substantially cover the cartridge (2).

### List of Drawing Signs

- 1: personal vaporizer device or e-cigarette
- 2: cartridge
- 3: end region of elongate body
- 4: elongate body
- 5: casing
- 6: cover member
- 6': front panel
- 6": rear panel
- 7: housing
- 7': housing base
- 7": housing sides
- 7''': housing shoulder region
- 8: reservoir
- 9: mouthpiece
- 10: vaporizer unit
- 14: electrical contact
- 15: conductor element
- 17: switch or button
- 18: opening in the cover member
- 19: ring-shaped indicator
- 21: mode switch or button
- 22: opposite end region of body portion
- 23: charging connector
- 24: sleeve element
- 25: rail member
- 26: circuit board
- 29: light-guide element
- 31: resilient or flexible member
- 32: projecting lug
- 33: recess
- 34: electrical contact
- 35: magnetic connector element
- A: first position of cover member
- B: second position of cover member
- D: battery unit
- C: airflow channel
- S: sides of the casing
- W: window

## Claims

1. A personal vaporizer (1), especially an electronic smoking article, comprising:
- an elongated body (4) having a casing (5) which encloses a power supply unit and a cover member (6) movable in a longitudinal direction of the elongated body (4) between a first position and a second position;
- a replaceable cartridge (2) attached to the elongated body (4) and comprising a housing (7) that encloses a reservoir (8) for storing a liquid to be vaporized,
wherein the cartridge (2) is at least partially covered or obscured by the cover member (6) in the first position (A) and wherein the cartridge (2) is removed and/or connected when the cover member is in the second position (B); and
wherein the personal vaporizer further includes a user interface having one or more LEDs for indicating an operational status of the device and/or for indicating available power supply in the power supply unit.

2. The personal vaporizer (1) according to claim 1, wherein the replaceable cartridge (2) comprises a vaporizer unit (10).

3. The personal vaporizer (1) according to claim 2, wherein the vaporizer unit (10) comprises a heater for heating the liquid to generate a vapour to be inhaled, and a liquid delivery means which is configured to convey the liquid from the reservoir (8) to the heater for vaporization.

4. The personal vaporizer (1) according to any one of the preceding claims, wherein the elongated body further comprises at least one part of a controller or a control unit configured to manage one or more of: a heater or heating function of the device, a power supply function of the device, and a capsule recognition function.

5. The personal vaporizer (1) according to any one of the preceding claims, wherein it further comprises one or more sensors for determining one or more operating conditions of the device.

6. The personal vaporizer (1) according to claim 5, wherein the one or more sensors are selected from the group consisting of a temperature sensor, a liquid volume sensor, a puff sensor or gyroscopic sensors.

7. The personal vaporizer (1) according to any one of the preceding claims, wherein the cover member (6) comprises a window (W) such that the volume or amount of liquid in the reservoir (8) of the replaceable cartridge (2) remains visible when the cover is in the first position (A).

8. The personal vaporizer (1) according to any one of the preceding claims, wherein the elongated body (4) comprises an end region (3) including one or more electrical contacts (14) for making connection with one or more complementary electrical connectors provided on the replaceable cartridge (2) for connecting electrical power to the heater of the vaporizer unit (10) within the replaceable cartridge (2).

9. The personal vaporizer (1) according to any one of the preceding claims, wherein the replaceable cartridge (2) is configured for a push-on/push-off type connection into an open upper end region (3) of the casing (5) of the device.

10. The personal vaporizer (1) according to any one of the preceding claims, wherein the replaceable cartridge (2) is configured for a mechanical connection effected via a magnetic connector element (35) provided on both the replaceable cartridge (2) and an end region (3) of the elongated body (4).

11. The personal vaporizer (1) according to any one of the preceding claims, wherein the replaceable cartridge (2) includes an airflow channel (C) configured to guide the vapour to a mouthpiece (9) for inhalation by a user.

12. The personal vaporizer (1) according to any one of the preceding claims, wherein the second position (B) of the cover member (6) is a substantially retracted position in which the replaceable cartridge (2) is substantially uncovered or unobscured by the cover member (6), for connection of the replaceable cartridge (2) to, and/or removal of the replaceable cartridge (2) from, the elongated body (4).

13. The personal vaporizer (1) according to any one of the preceding claims, wherein the cover member (6) forms a part of the outer casing (5) of the elongated body (4).

14. The personal vaporizer (1) according to any one of the preceding claims, wherein the elongated body (4) comprises a switch (17) operatively connected with the a part of the controller or control unit and configured to enable at least one functionality when the cover member (6) is moved to the first position (A) and/or to the second position (B).

15. The personal vaporizer (1) according to any one of the preceding claims, wherein the power supply unit is formed as a battery unit (D) and/or the one or more LEDs act or serve as illuminated indicator elements.

16. The personal vaporizer (1) according to any one of the preceding claims, wherein the replaceable cartridge (2) is attached to the elongated body (4) at one end region of the elongated body.
